# EUROPEAN PATENT SPECIFICATION

(11) **EP 0 836 612 B1**
(45) Date of publication and mention of the grant of the patent: **26.07.2000**
(21) Application number: 96920360.3
(22) Date of filing: 23.05.1996
(51) Int. Cl.: C07H 19/11, C07H 19/213, C07H 21/00

(54) **NOVEL SYNTHONS FOR STEREOSELECTIVE OLIGONUCLEOTIDE SYNTHESIS**
SYNTHON FÜR STEREOSELEKTIVE OLIGONUKLEOTID-SYNTHESE
SYNTHONS D'UN TYPE NOUVEAU PERMETTANT LA SYNTHESE STEREOSELECTIVE D'OLIGONUCLEOTIDES

(30) Priority: 23.05.1995 US 447384; 23.05.1995 US 447494; 23.05.1995 US 448131; 23.05.1995 US 448632; 01.06.1995 US 457198; 08.02.1996 US 597434
(43) Date of publication of application: 22.04.1998
(73) Proprietor: HYBRIDON, INC., Milford, MA 01757 (US)
(72) Inventor: IYER, Radhakrishnan, P., Shrewsbury, MA 01545 (US); DEVLIN, Theresa, Jamaica Plain, MA 02130 (US); HABUS, Ivan, Shrewsbury, MA 01545 (US); YU, Dong, Shrewsbury, MA 01545 (US); AGRAWAL, Sudhir, Shrewsbury, MA 01545 (US)
(74) Representative: Vossius, Volker, Dr.
(86) International application number: US9607386
(87) International publication number: WO9637504

(56) References cited:
- US-A- 5 212 295
- JOURNAL OF THE CHEMICAL SOCIETY, PERKIN TRANSACTIONS 1, 1993, LETCHWORTH GB, pages 1699-1704, XP002016241 KRASZEWSKI A. ET AL: "Studies on Reactions of Nucleoside H-Phosphonates with Bifunctional Reagents. Part 1. Reaction with amino alcohols"
- JOURNAL OF THE CHEMICAL SOCIETY, PERKIN TRANSACTIONS 1, 1985, LETCHWORTH GB, pages 199-202, XP002016242 JONES S. ET AL: "Synthesis of some Nucleoside Cyclic Phosphoramidates and Related compounds via Phosphoramidites"
- TETRAHEDRON: ASYMMETRY, vol. 6, no. 5, 26 May 1995, OXFORD GB, pages 10941-1054, XP002016243 IYER R.P. ET AL: "A Novel Nucleoside Phosphoramidite Synthon Derived from 1R,2S-Ephedrine"
- JOURNAL OF ORGANIC CHEMISTRY, vol. 60, December 1995, EASTON US, pages 5388-5389, XP002016244 IYER R.P. ET AL: "Nucleoside oxazaphospholidines as Novel Synthons in Oligonucleotide synthesis"

## Description

The invention relates to the chemical synthesis of oligonucleotides and to chemical entities useful in such synthesis.

### Summary of the Related Art

Oligonucleotides have become indispensible tools in modern molecular biology, being used in a wide variety of techniques, ranging from diagnostic probing methods to PCR to antisense inhibition of gene expression. This widespread use of oligonucleotides has led to an increasing demand for rapid, inexpensive and efficient methods for synthesizing oligonucleotides.

The synthesis of oligonucleotides for antisense and diagnostic applications can now be routinely accomplished. See *e.g.,Methods in Molecular Biology, Vol 20: Protocols for Oligonucleotides and Analogs* pp. 165-189 (S. Agrawal, Ed., Humana Press, 1993); *Oligonucleotides and Analogues: A Practical Approach,* pp. 87-108 (F. Eckstein, Ed., 1991); and Uhlmann and Peyman, *supra.* Agrawal and Iyer, *Curr. Op. in Biotech.* **6,** 12 (1995); and *Antisense Research and Applications* (Crooke and Lebleu, Eds., CRC Press, Boca Raton, 1993). Early synthetic approaches included phosphodiester and phosphotriester chemistries. Khorana et al., *J. Molec. Biol.* **72,** 209 (1972) discloses phosphodiester chemistry for oligonucleotide synthesis. Reese, *Tetrahedron Lett.* **34**, 3143-3179 (1978), discloses phosphotriester chemistry for synthesis of oligonucleotides and polynucleotides. These early approaches have largely given way to the more efficient phosphoramidite and H-phosphonate approaches to synthesis. Beaucage and Caruthers, *Tetrahedron Lett.* **22,** 1859-1862 (1981), discloses the use of deoxynucleoside phosphoramidites in polynucleotide synthesis. Agrawal and Zamecnik, U.S. Patent No. 5,149,798 (1992), discloses optimized synthesis of oligonucleotides by the H-phosphonate approach.

Both of these modern approaches have been used to synthesize oligonucleotides having a variety of modified internucleotide linkages. Agrawal and Goodchild, *Tetrahedron Lett.* **28**, 3539-3542 (1987), teaches synthesis of oligonucleotide methylphosphonates using phosphoramidite chemistry. Connolly et al., *Biochemistry* **23,** 3443 (1984), discloses synthesis of oligonucleotide phosphorothioates using phosphoramidite chemistry. Jager el al., *Biochemistry* **27***,* 7237 (1988), discloses synthesis of oligonucleotide phosphoramidates using phosphoramidite chemistry. Agrawal et al., *Proc. Antl. Acad. Sci. USA* **85**, 7079-7083 (1988), discloses synthesis of oligonucleotide phosphoramidates and phosphorothioates using H-phosphonate chemistry.

Solid phase synthesis of oligonucleotides by each of the foregoing methods involves the same generalized protocol. Briefly, this approach comprises anchoring the 3'-most nucleoside to a solid support functionalized with amino and/or hydroxyl moieties and subsequently adding the additional nucleosides in stepwise fashion. Desired internucleoside linkages are formed between the 3' functional group of the incoming nucleoside and the 5' hydroxyl group of the 5'-most nucleoside of the nascent, support-bound oligonucleotide.

Refinement of methodologies is still required, however, particularly when making a transition to large-scale synthesis (lOumol to 1 mmol and higher). See Padmapriya et al., *Antisense Res. Dev.* **4**, 185 (1994). Several modifications of the standard phosphoramidite methods have already been reported to facilitate the synthesis (Padmapriya et al., *supra;* Ravikumar et al., *Tetrahedron* **50**, 9255 (1994); Theisen et al., *Nucleosides & Nucleotides* **12***,* 43 (1994); and Iyer et al., *Nucleosides & Nucleotides* **14**, 1349 (1995)) and isolation (Kuijpers et al. *Nucl. Acids Res.* 18, 5197 (1990); and Reddy et al., *Tetrahedron Lett.* 35, 4311 (1994)) of oligonucleotides.

A. Kraszewski et al., *J. Chem. Soc. Perkin Trans. I* **1993,** 1699, describes the reaction of nucleoside *H*-phosphonates with amino alcohols in the presence of condensing agents. Depending on the coupling procedure used and the length of the polymethylene bridge in the amino alcohols, different *H*-phosphonate diesters, cyclic phosphoramidite derivatives or phosphite triesters can be produced. Oxidation of these species with iodine under various experimental conditions has been studied and a general procedure for synthesis of nucleoside alkyl phosphodiesters has been developed.

S. Jones et al., *J. Chem. Soc. Perkin Trans. I* **1985**, 199, describes the reaction of 2-chloro-3-methyl-1-oxa-3-aza-2-phosphacyclopentane with thymidine and with 2'-deoxy-5-fluorouridine, which resulted in the formation of their 3',5'-bis-*O*-(3-methyl-1-oxa-3-aza-2-phosphacyclopentan-2-yl) derivatives. Oxidation of these derivatives with dinitrogen tetraoxide resulted in opening of the phosphoramidate ring.
Reaction of the above nucleosides with 2-chloro-1,3-dimethyl-1,3-diaza-2-phosphacyclopentane gave their 3',5'-bis-*O*-(1,3-dimethyl-1,3-diaza-2-phosphacyclopentan-2-yl) derivatives, which were oxidised with dinitrogen tetraoxide to the corresponding phosphoramidates.

### BRIEF SUMMARY OF THE INVENTION

The invention provides new reagents and processes for synthesizing oligonucleotides, including stereoselective oligonucleotide synthesis.

In a first aspect, the invention provides novel monomer synthons for the synthesis of oligonucleotides. Novel monomer synthons according to the invention are characterized by the general structure I: 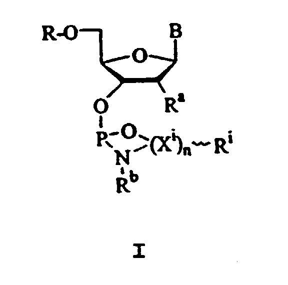 wherein R^{a} and R^{b}, and each Rⁱ are independently H or a C₁-C₂₀ alkyl, aryl, heterocyclic or alkoxy group, R is a suitable protecting group, n is 1-3, i is 1-n, Xⁱ is C, O, S, or N, such that if n > 1 the identity of each Xⁱ (*i.e.*, each of X¹...Xⁿ) is independent of the identity of every other Xⁱ and the identity of each substituent Rⁱ (*i.e*., each of R¹...Rⁿ) is independent of every other Rⁱ, each Rⁱ is covalently bound to the corresponding Xⁱ (*e.g.*, X¹-R¹ ...Xⁿ-Rⁿ), the Xⁱ are arranged consecutively such that X¹ is bound to the N and Xⁿ is bound to the O, and B is any suitably protected modified or unmodified purine or pyrimidine base, with the proviso, that if B = thymine and R = dimethoxytrityl, R^{a} and R^{b} are not each H. In one preferred embodiment of a monomer synthon according to this aspect of the invention, R^{a} is H, n is 2 and X¹ and X² are each C, with the proviso, that if B = thymine and R = dimethoxytrityl, R^{b} is not H. In a particularly preferred embodiment of a monomer synthon according to this aspect of the invention, n is 2, X¹ and X² are each C, R¹ is methyl, R² is phenyl, R^{a} is H, R^{b} is methyl, and the synthon has the Rₚ configuration. In another particularly preferred embodiment of a monomer synthon according to this aspect of the invention, n is 2, X¹ and X² are each C, R¹ and R² are each H, R^{a} is H, and R^{b} is methyl.

Certain preferred monomer synthons according to this aspect of the invention are pentavalent at the phosphorous atom. These preferred monomer synthons are characterized by the general structure **V**: 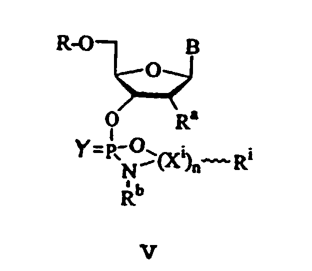 wherein Y is sulfur or an isotope of oxygen, R^{a} and R^{b}, and each Rⁱ are independently H or a C₁-C₂₀ alkyl, aryl, heterocyclic or alkoxy group, R is a suitable protecting group, n is 1-3, i is 1-n, Xⁱ is C, O, S, or N, such that if n > 1 the identity of each Xⁱ (*i.e.,* each of X¹...Xⁿ) is independent of the identity of every other Xⁱ and the identity of each substituent Rⁱ (*i.e.,* each of R¹...Rⁿ) is independent of every other Rⁱ, each Rⁱ is covalently bound to the corresponding Xⁱ (*e.g.*, X¹-R¹...Xⁿ-Rⁿ), the Xⁱ are arranged consecutively such that X¹ is bound to the N and Xⁿ is bound to the O, and B is any suitably protected modified or unmodified purine or pyrimidine base, with the proviso, that if B = thymine and R = dimethoxytrityl, R^{a} and R^{b} are not each H. In one preferred embodiment of a monomer synthon according to the general structure **V**, R^{a} is H, n is 2 and X¹ and X² are each C_{,} with the proviso, that if B = thymine and R = dimethoxytrityl, R^{b} is not H. In a particularly preferred embodiment of a monomer synthon according to this structure, n is 2, X¹ and X² are each C, R¹ is methyl, R² is phenyl, R^{a} is H, R^{b} is methyl, and the synthon may be the *anti*-isomer or the *syn*-isomer. In another particularly preferred embodiment of a monomer synthon according to structure **VI,** n is 2, X¹ and X² are each C, R¹ and R² are each H, R^{a} is H, and R^{b} is methyl.

Monomer synthons according to this aspect of the invention are useful in the synthesis of oligonucleotides and can be used in place of the well known beta-cyanoethyl phosphoramidite monomer synthon in the phosphoramidite synthesis procedure. Certain monomer synthons according to this aspect of the invention are useful in this procedure for producing oligonucleotides having defined stereochemistry.

In a second aspect, the invention provides processes for synthesizing monomer synthons according to the invention, including diastereomerically enriched or purified monomer synthons. In the processes according to this aspect of the invention, the chemical reactions are stereoretentive so that the products of each reaction retain the same stereoconfiguration as their precursor reagent. In the most general process according to this aspect of the invention, the reagent PCl₃ is reacted with a compound having the structure **X**: 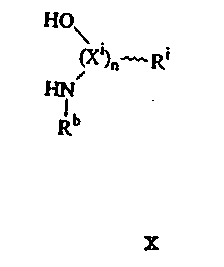 wherein R^{b} and each Rⁱ are independently H or a C₁-C₂₀ alkyl, aryl, heterocyclic or alkoxy group, n is 1-3, i is 1n, Xⁱ is C, O, S, or N, such that if n > 1 the identity of each Xⁱ (*i.e.*, each of X¹...Xⁿ) is independent of the identity of every other Xⁱ and the identity of each substituent Rⁱ (*i.e*., each of R¹ ...Rⁿ) is independent of every other Rⁱ, each Rⁱ is covalently bound to the corresponding Xⁱ (*e.g.*, X¹-R¹...Xⁿ-Rⁿ), and the Xⁱ are arranged consecutively such that X¹ is bound to the N and Xⁿ is bound to the O,
to yield the structure **XI**: 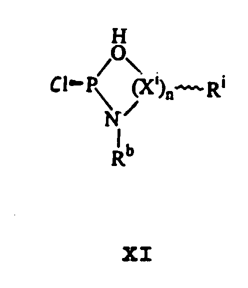 which is then reacted with a nucleoside having a protected 5' hydroxyl and unprotected 3' hydroxyl to yield the previously described monomer synthon structure **I**. In a preferred embodiment of this process according to the invention, in the compound having the structure **X**, n is 2 and X¹ and X² are each C. In a particularly preferred embodiment of the process according to this aspect of the invention, the process comprises the step of reacting a pure stereoisomer of ephedrine with PCl₃. When the stereoisomer (1R, 2S)-(-)-ephedrine is used, the result is a yield of about 75% of a chlorophosphoramidite product which is 95% in the Rₚ isomer configuration. This compound reacts with a nucleoside having a protected 5' hydroxyl and unprotected 3' hydroxyl to yield a stereoregular monomer synthon. The other stereoisomers of ephedrine (1S,2R; 1S,2S; and 1R,2R) can be used in place of (1R,2S)-(-)-ephedrine to obtain their corresponding stereoregular monomer synthons. In another preferred embodiment of the process according to this aspect of the invention, in the compound having structure **X**, X¹ and X² are each C, R¹ and R² are each H, and R^{b} is CH₃.

In another preferred embodiment of a process according to this aspect of the invention, the previously described monomer synthons having structure **I**, or the previously described particularly preferred embodiments thereof, are oxidized stereoretentively to yield respectively the previously described monomer synthon structure **V**, or the previously described particularly preferred embodiments thereof.

In another preferred embodiment, the process according to this aspect of the invention can be used to provide both the *anti*- and *syn*- isomer synthons for stereoselective oligonucleotide synthesis. The isomers are then readily separated by conventional chromatography or crystallization. In another preferred embodiment, the process according to this aspect of the invention can be used to produce a new monomer synthon for nonstereoselective synthesis of phosphorothioate or phosphodiester oligonucleotides.

In any of the above preferred embodiments of the process according to this aspect of the invention, the oxidation can be an oxidative thiolation of the phosphorous, and the constituent Y of the compound **V** thereby produced is sulfur. Alternatively, the oxidation can be an oxygenation of the phosphorous, and the constituent Y of the compound **V** thereby produced is oxygen, most preferably an isotope of oxygen other than ¹⁸O.

In a third aspect, the invention provides processes for synthesizing oligonucleotides using the well known phosphoramidite approach. In the processes according to this aspect of the invention, any of the monomer synthons according to the invention is used in place of the conventional beta-cyanoethyl phosphoramidite.

In one preferred embodiment, synthon **I** or one of its particularly preferred embodiments is used in the synthesis process. The synthon is contacted in the presence of a suitable activating agent, such as tetrazole, with a nascent oligonucleotide (including a support-bound mononucleoside) having a free 5' hydroxyl to form a phosphite internucleoside linkage. The phosphite internucleoside linkage is then oxidized using a suitable oxidizing agent. When a chiral preferred embodiment of synthon **I** is used in the synthesis process, the resulting oligonucleotides generally have predominantly Sₚ configuration at each internucleoside linkage at which such chiral synthon was used during synthesis.

In another preferred embodiment, synthon **V** or one of its particularly preferred embodiments is used in the synthesis process. The synthon is contacted in the presence of a suitable activating agent, such as tetrazole, with a nascent oligonucleotide (including a support-bound mononucleoside) having a free 5' hydroxyl. In this case, the synthon has already been oxidized at the phosphorous atom, so the resulting internucleoside linkage is a phosphodiester or phosphorothioate linkage. When a chiral preferred embodiment of synthon **V** is used and constituent Y is sulfur or an isotope of oxygen other than ¹⁸O, the resulting oligonucleotide has a Rₚ :Sₚ ratio of about 70:30 or 10:90, depending on which epimer of **V** is used.

The process according to this aspect of the invention utilizes phosphoramidite chemistry for any cycles in which any of the monomer synthons according to the invention are used. However, in other cycles, the process according to this aspect of the invention can utilize any suitable oligonucleotide synthesis chemistry, including the well known H-phosphonate, phosphotriester and phosphoramidite chemistries.

### DETAILED DESCRIPTION OF THE PREFERRED EMBODIMENTS

The invention relates to the chemical synthesis of oligonucleotides and to chemical entities useful in such synthesis. The patents and publications identified in this specification are within the knowledge of those skilled in this field.

The invention provides new reagents and processes for synthesizing oligonucleotides, including stereoselective oligonucleotide synthesis.

In a first aspect, the invention provides novel monomer synthons for the synthesis of oligonucleotides. Novel monomer synthons according to the invention are characterized by the general structure **.I**: 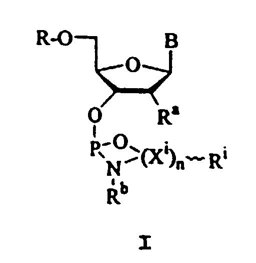 wherein R^{a} and R^{b}, and each Rⁱ are independently H or a C₁-C₂₀ alkyl, aryl, heterocyclic or alkoxy group, R is a suitable protecting group (see Sonveaux in *Methods in Molecular Biology, Vol. 26: Protocols for Oligonucleotide Conjugates (Agrawal, Ed.)(1994),* Humana Press Inc., Totowa, NJ), n is 1-3, i is 1-n, Xⁱ is C, O, S, or N, such that if n > 1 the identity of each Xⁱ (*i.e*., each of X¹...Xⁿ) is independent of the identity of every other Xⁱ and the identity of each substituent Rⁱ (*i.e.,* each of R¹...Rⁿ) is independent of every other Rⁱ, each Rⁱ is covalently bound to the corresponding Xⁱ (*e.g.,* X¹-R¹...Xⁿ-Rⁿ), the Xⁱ are arranged consecutively such that X¹ is bound to the N and Xⁿ is bound to the O, and B is any suitably protected modified or unmodified purine or pyrimidine base, with the proviso, that if B = thymine and R = dimethoxytrityl, R^{a} and R^{b} are not each H. (see Sonveaux, *supra* and Meyer, *Methods in Molecular Biology,* Vol. 26, pp. 73-92). As used throughout this disclosure, the term "aryl" means a polyaromatic ring structure having from 1 to 5 linearly or angularly fused aromatic rings, such as phenyl and napthyl. As used throughout this disclosure, the term "heterocyclic" means a 5 or 6 membered ring having from 1 to 5 heteroatoms (*i.e.*, N, S, or O) which may be located at any position within the ring, examples of which include furan and thiophene.

In one preferred embodiment of a monomer synthon according to this aspect of the invention, R^{a} is H, n is 2 and X¹ and X² are each C, with the proviso, that if B = thymine and R = dimethoxytrityl, R^{b} is not H resulting in the structure **II**: 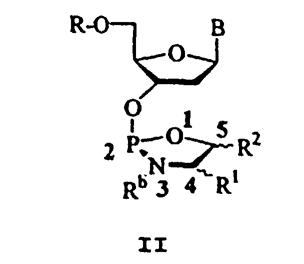 In this preferred embodiment, the configurations at carbons 4 and 5 can be, respectively, R and S, S and R, R and R, or S and S, each of which can be obtained in pure form.

In a particularly preferred embodiment of a monomer synthon according to this aspect of the invention, n is 2, X¹ and X² are each C, R¹ is methyl, R² is phenyl, R^{a} is H, R^{b} is methyl, and the synthon has the Rₚ configuration shown in structure **III**:

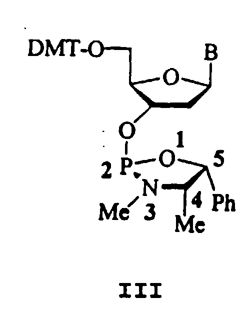

In another particularly preferred embodiment of a monomer synthon according to this aspect of the invention, n is 2, X¹ and X² are each C, R¹ and R² are each H, R^{a} is H, R^{b} is methyl, and the synthon is the phosphoramidite synthon shown in structure **IV**:

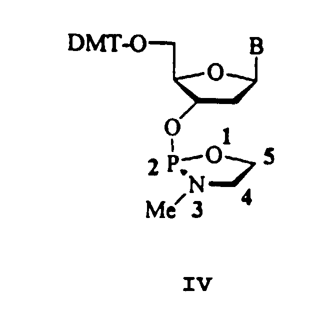

Certain preferred monomer synthons according to this aspect of the invention are pentavalent at the phosphorous atom. These preferred monomer synthons are characterized by the general structure **V**: 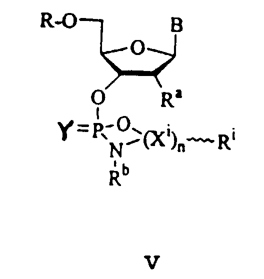 wherein Y is sulfur or an isotope of oxygen, R^{a} and R^{b}, and each Rⁱ are independently H or a C₁-C₂₀ alkyl, aryl, heterocyclic or alkoxy group, R is a suitable protecting group, n is 1-3, i is 1-n, Xⁱ is C, O, S, or N, such that if n > 1 the identity of each Xⁱ (*i.e.*, each of X¹...Xⁿ) is independent of the identity of every other Xⁱ and the identity of each substituent Rⁱ (*i.e.*; each of R¹...Rⁿ) is independent of every-other Rⁱ, each Rⁱ is covalently bound to the corresponding Xⁱ (*e.g*., X¹-R¹...Xⁿ-Rⁿ), the Xⁱ are arranged consecutively such that X¹ is bound to the N and Xⁿ is bound to the O, and B is any suitably protected modified or unmodified purine or pyrimidine base, with the proviso, that if B = thymine and R = dimethoxytrityl, R^{a} and R^{b} are not each H.

In one preferred embodiment of a monomer synthon according to the general structure **V**, R^{a} is H, n is 2 and X¹ and X² are each C, with the proviso, that if B = thymine and R = dimethoxytrityl, R^{b} is not H resulting in the structure **VI**:

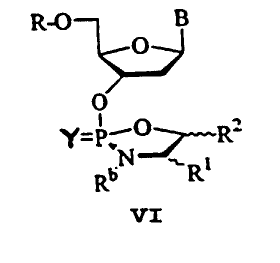

In a particularly preferred embodiment of a monomer synthon according to structure **VI**, n is 2, X¹ and X² are each C, R¹ is methyl, R² is phenyl, R^{a} is H, R^{b} is methyl, and the synthon may be the *anti*-isomer shown in structure VII or the *syn*-isomer shown in structure **VIII**:

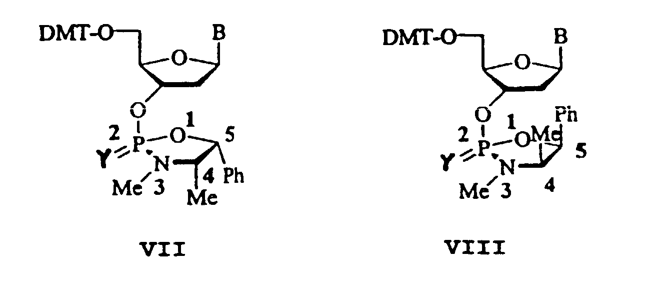

In another particularly preferred embodiment of a monomer synthon according to structure **VI**, n is 2, X¹ and X² are each C, R¹ and R² are each H, R^{a} is H, R^{b} is methyl, and the synthon is the phosphoramidite synthon shown in structure **IX**:

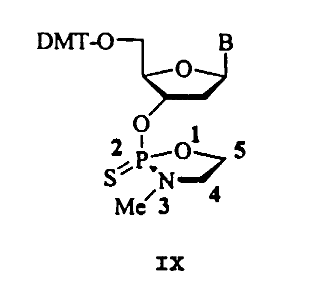

Monomer synthons according to this aspect of the invention are useful in the synthesis of oligonucleotides and can be used in place of the well known beta-cyanoethyl phosphoramidite monomer synthon in the phosphoramidite synthesis procedure (see *e.g.,* Beaucage in *Methods in Molecular Biology,* Vol. 20, *Protocols for Oligonucleotides and Analogs,* pp. 33-61). Certain synthons having the structures **III** and **V-VIII** are useful in this procedure for producing oligonucleotides having defined stereochemistry.

In a second aspect, the invention provides processes for synthesizing monomer synthons according to the invention, including diastereomerically enriched or purified monomer synthons. In the processes according to this aspect of the invention, the chemical reactions are stereospecific so that the products of each reaction possess a defined stereoconfiguration. In the most general process according to this aspect of the invention, the reagent PCl₃ is reacted with a compound having the structure **X**: 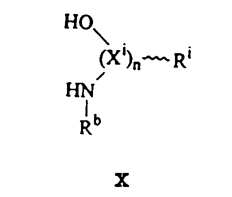 wherein R^{b} and each Rⁱ are independently H or a C₁-C₂₀ alkyl, aryl, heterocyclic or alkoxy group, n is 1-3, i is 1n, Xⁱ is C, O, S, or N, such that if n > 1 the identity of each Xⁱ (*i.e.*, each of X¹ ...Xⁿ) is independent of the identity of every other Xⁱ and the identity of each substituent Rⁱ (*i.e.*, each of R¹...Rⁿ) is independent of every other Rⁱ, each Rⁱ is covalently bound to the corresponding Xⁱ (*e.g.*, X¹-R¹...Xⁿ-Rⁿ), and the Xⁱ are arranged consecutively such that X¹ is bound to the N and Xⁿ is bound to the O,
to yield the structure **XI**: 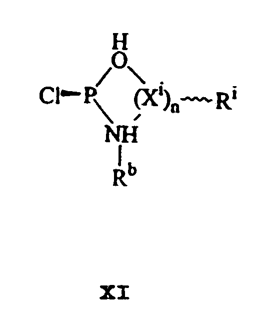 which is then reacted with a nucleoside having a protected 5' hydroxyl and unprotected 3' hydroxyl to yield the previously described monomer synthon structure **I**.

In a preferred embodiment of this process according to the invention, in the compound having the structure **X**, n is 2 and X¹ and X² are each C, resulting in the structure **XII**, 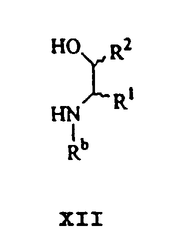 which, when reacted with PCl₃, produces the structure **XIII**: 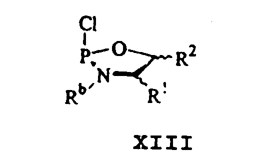 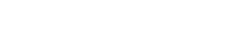 which is then reacted with a nucleoside having a protected 5' hydroxyl and unprotected 3' hydroxyl to yield the previously described monomer synthon structure **II**.

In a particularly preferred embodiment of the process according to this aspect of the invention, the process comprises the step of reacting a pure stereoisomer of ephedrine with PCl₃, preferably at a temperature between minus 100 and +40° C for a time between one and 40 hours, most preferably in N-methyl morpholine and toluene at -78 °C for three hours and then 22 °C for 12 hours. Other suitable solvents are benzene, tetrahydrofuran, ether and dioxane. When the stereoisomer (1R, 2S)-(-)-ephedrine is used, the result is a yield of about 75% of a chlorophosphoramidite product having the structure **XIV**, which is 95% in the Rₚ isomer configuration: 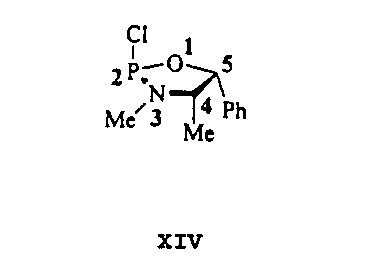 This compound is fairly stable, undergoing no decomposition detectable by ³¹P-NMR after being stored at -5 °C for several days. It reacts with a nucleoside having a protected 5' hydroxyl and unprotected 3' hydroxyl to yield the previously described monomer synthon structure **III** in high yield (84%). Most preferably, the reaction with the nucleoside is carried out in the presence of a scavenger of the HC1 liberated during the reaction, such as triethylamine, pyridine, and 2,6-lutidine. The other stereoisomers of ephedrine (1S,2R; 1S,2S; and 1R,2R) are also commercially available and can be used in place of (1R,2S)-(-)-ephedrine to obtain, respectively, compounds having structures **XV, XVI**, and **XVII:** 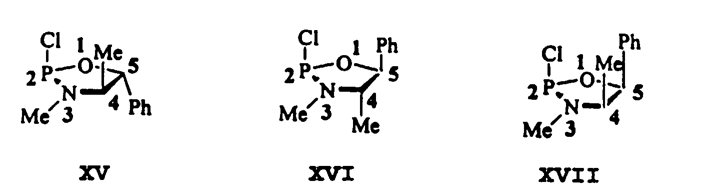 any of which reacts with a nucleoside having a protected 5' hydroxyl and unprotected 3' hydroxyl to yield the corresponding other stereoisomer of the previously described monomer synthon structure **III.**

In another particularly preferred embodiment of the process according to this aspect of the invention, the process comprises the step of reacting a compound having the structure **XVIII**: 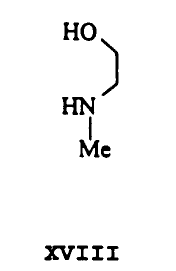 with PCl₃ to yield a compound having the structure **XIX:** 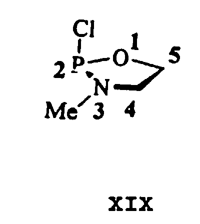 which reacts with a nucleoside having a protected 5' hydroxyl and unprotected 3' hydroxyl to yield the previously described monomer synthon structure **IV.**

In another preferred embodiment of a process according to this aspect of the invention, monomer synthons having the previously described structure **I** or **II** are oxidized stereoretentively to yield respectively the previously described monomer synthon structure **V** or **VI**. Stereospecific oxidation of a particular stereoisomer of compound **I** or **II** results in the oxidation product in approximatley 90% yield.

In another preferred embodiment, the process according to this aspect of the invention can be used to provide the anti- isomer synthon previously described as structure **VII**. In this embodiment, the previously described synthon **III** is oxidized to yield a mixture containing 90% *anti-* isomer **VII** and 10% *syn*- isomer **VIII**. Synthons **VII** and **VIII** are then readily separated by conventional chromatography or crystallization.

In another preferred embodiment, the process according to this aspect of the invention can be used to provide the synthon previously described as structure **IX**. In this embodiment, the previously described synthon **IV** is oxidized to yield the synthon **IX**.

In any of the above preferred embodiments of the process according to this aspect of the invention, the oxidation can be an oxidative thiolation of the phosphorous, and the constituent Y of the compound **V-IX** thereby produced is sulfur. Such thiolation may be carried out using any suitable thiolating agent, such as elemental sulfur. (See Stec *et al.,* J. Am. Chem. Soc. 106: 6077 (1984).) Preferably, such oxidative thiolation is carried out using Beaucage reagent, 3H-1,2-benzodithiol-3-one-1,1-dioxide. (See Iyer *et al.,* J. Am. Chem. Soc. 112: 1253 (1990) and Iyer *et al., J.* Org. Chem. 55: 4693 (1990).) In a most preferred embodiment, Beaucage reagent is used as a 2% solution in acetonitrile and is allowed to react for about thirty seconds at about room temperature with any of compounds **I-IV**. Alternatively, the oxidation can be an oxygenation of the phosphorous, and the constituent Y of the compound **V-IX** thereby produced is oxygen, most preferably an isotope of oxygen other than ¹⁸O. In this embodiment, the oxidation is carried out using a suitable oxidizing agent in the presence of water, preferably in the presence of water having an isotope of oxygen other than ¹⁸O, such as ¹⁷O. Suitable oxidizing agents include, without limitation, I₂, tert-BuOOH and N₂O₄ (see Beaucage and Iyer, Tetrahedron 48: 2223 (1992)). Other chiral constituents include, without limitation, selenium and tellurium.

In a third aspect, the invention provides processes for synthesizing oligonucleotides using the well known phosphoramidite approach. (See Beaucage in *Methods in Molecular Biology, Vol.* 20, *Protocols for Oligonucleotides and Analogs, supra,* at pp. 33-61.) In the processes according to this aspect of the invention, any of synthons **I-IX** is used in place of the conventional beta-cyanoethyl phosphoramidite.

In one preferred embodiment, any of synthons **I-IV** are used in the synthesis process. The synthon is contacted in the presence of a suitable activating agent with a nascent oligonucleotide (including a support-bound mononucleoside) having a free 5' hydroxyl to form a phosphite internucleoside linkage. Suitable activating agents include, without limitation, tetrazole, anilinium trifluoroacetate, substituted tetrazole derivatives (*e.g.,* phenyl or thioethyltetrazole) and N,N-dimethylaniline hydrochloride (see Beaucage and Iyer, *supra).* The phosphite internucleoside linkage is then oxidized using a suitable oxidizing agent. For example, it may be oxidized using I₂ and H₂O in THF to yield a phosphodiester internucleoside linkage. If H₂O containing an isotope of oxygen other than ¹⁸O is used, the phosphodiester internucleoside linkage will be isotopically labeled. Alternatively, the phosphite internucleoside linkage may be oxidized using a thiolating agent, such as S₈ or Beaucage reagent, to yield a phosphorothioate internucleoside linkage. When compound **III** is used in the synthesis process, the resulting phosphorothioate oligonucleotides or isotopically labeled phosphodiester oligonucleotides have predominantly Sₚ configuration (about 60%) at each internucleoside linkage at which compound **III** was used during synthesis.

In another preferred embodiment, any of synthons **V-IX** are used in the synthesis process. The synthon is contacted in the presence of a suitable activating agent with a nascent oligonucleotide (including a support-bound mononucleoside) having a free 5' hydroxyl. Suitable activating agents include, without limitation, hindered non-nucleophilic bases, such as 1,4-diazabicycloundecene, potassium tert-butoxide and t-butyl magnesium chloride. In this case, the synthon has already been oxidized at the phosphorous atom, so the resulting internucleoside linkage is a phosphodiester or phosphorothioate linkage. When compound **VII** is used and constituent Y is sulfur or an isotope of oxygen other than ¹⁸O, the resulting oligonucleotide has a Rₚ :Sₚ ratio of about 70:30. When compound **VIII** is used, the resulting oligonucleotide has a Rₚ :Sₚ ratio of 10:90. Similar results are obtained using compound **V**, when constituent Y is sulfur or an isotope of oxygen other than ¹⁸O and all of the Rⁱ are *anti-* or *syn*-with respect to the nucleoside, or using compound **VI**, when constituent Y is sulfur or an isotope of oxygen other than ¹⁸O and R¹ and R² are both *anti*- or both *syn*- with respect to the nucleoside.

The process according to this aspect of the invention utilizes phosphoramidite chemistry for any cycles in which any of compounds **I-IX** are used. However, in other cycles, the process according to this aspect of the invention can utilize any suitable oligonucleotide synthesis chemistry, including the well known H-phosphonate, phosphotriester and phosphoramidite chemistries. In one preferred embodiment, synthesis is carried out on a suitable solid support. Suitable solid supports include any of the standard solid supports used for solid phase oligonucleotide synthesis, such as controlled-pore glass (CPG). (See, *e.g.*, Pon, Methods in Molec. Biol. 20: 465 (1993)). Synthesis on such a solid support begins with coupling a nucleoside synthon according to the invention to a nucleoside that is covalently linked the solid support (*i.e.*, linked to a functionality on the solid support, preferably an amino or hydroxyl functionality).

The versatility of chemical synthetic approach of the method according to the invention makes the method according to the invention suitable for the synthesis of any of a broad class of compounds, all of which are referred to herein as "oligonucleotides". For purposes of the invention, the term oligonucleotide includes polymers of two or more deoxyribonucleotide, or 2'-O-substituted ribonucleotide monomers, or any combination thereof. Such monomers may be coupled to each other by any of the numerous known internucleoside linkages. In certain preferred embodiments, these internucleoside linkages may be phosphodiester, phosphotriester, phosphorothioate, or phosphoramidate linkages, or combinations thereof. The term oligonucleotide also encompasses such polymers having chemically modified bases or sugars and/ or having additional substituents, including without limitation lipophilic groups, intercalating agents, diamines and adamantane. For purposes of the invention the term "2'-O-substituted" means substitution of the 2' position of the pentose moiety with an -O-lower alkyl group containing 1-6 saturated or unsaturated carbon atoms, or with an -O-aryl or allyl group having 2-6 carbon atoms, wherein such alkyl, aryl or allyl group may be unsubstituted or may be substituted, *e.g.*, with halo, hydroxy, trifluoromethyl, cyano, nitro, acyl, acyloxy, alkoxy, carboxyl, carbalkoxyl, or amino groups; or such 2' substitution may be with a hydroxy group(to produce a ribonucleoside), an amino or a halo group, but not with a 2'-H group.

The following examples further illustrate certain preferred embodiments of the invention and are not limiting in nature. Unless otherwise stated, all chemicals recited in the following Examples were obtained from Aldrich of Milwaukee, WI.

### Example 1

### Stereoselective Synthesis of a Mononucleotide Synthon

The chlorophosphoramidite, (2R,4S,5R)-2-chloro-3,4-dimethyl-5-phenyl-1,3,2-oxazaphospholidine (**XIV**) was obtained by mixing 8.14 g of 1R,2S-ephedrine and 10.4 ml of N-methyl morpholine in 250 ml of toluene under argon and cooling to -78°C. 4.3 ml of PCl₃ in 10 ml of toluene was added over a period of 15 minutes. The mixture was kept at - 78°C for 1 hour and then allowed to warm to room temperature over a period of 16 hours. The insoluble salt precipitate was filtered under argon. The precipitate was washed with 3 x 25 ml of toluene. The combined washings and filtrate were concentrated *in vacuo* in a rotary evaporator to remove toluene. Vacuum distillation of the residue gave a colorless liquid boiling at 0.1 mm Hg at 95°C to give ca. 9 g (80% yield) of the product. This procedure is similar to that described previously. Sun et al., *J. Chem. Soc. Perkin Trans. I,* p. 3183 (1994) and references therein and Carey et al., *J. Chem. Soc. Perkin Tarns. I,* p. 831 (1993).

³¹P-NMR examination of the resulting crude reaction mixture revealed the presence of a predominant isomer (> 95%) at δ 169.4 ppm and a minor component (< 5%) at δ 161 ppm. Upon vacuum distillation of the reaction mixture (95-97°C at 0.1 mm Hg), a colorless liquid was obtained, which solidified to a white crystalline mass upon cooling to -78°C (isolated yields of 75%). Carey et al, *supra,* reported a b.p. of 160°C at 0.1 mm Hg. NMR analysis gave the following results: ³¹P-NMR (CDCl₃) (TMP external standard) δ 169.1 ppm; ¹H-NMR (CDCl₃) δ (ppm) 0.71 (3H, d, J = 6.3 Hz), 2.69 (3H₃, d, ³J_{P-H} = 15.1 Hz, N-CH₃), 3.63 (1H, ddq, J = 1.3, 5.5, ³JP-H = 7.6 Hz, H-4), 5.85 (1H, dd, J = 5.5 Hz, ³J_{P-H} ~ 1.2 Hz), 7.15 (5H, m, -Ph). These spectral features are in agreement with values reported by Sun et al. and Carey et al., *supra,* and lead to the assignment of structure **XIV** as being the R isomer in which the chlorine atom is disposed *trans* relative to the C-Ph and C-Me substituents in the phospholidine ring. **XIV** could be stored as a solid in a desiccator at -5°C for several days with no apparent decomposition (as evaluated by ³¹P-NMR). Upon addition of water to **XIV**, the H-phosphonate was obtained as a mixture of diastereomers (Rₚ :Sₚ, 55:45 ³¹P-NMR).

2.16 g of 5'-O-dimethoxytrityl thymidine was dissolved in a mixture of anhydrous ether (20 ml) and anhydrous triethylamine (5 ml). The solution was added gradually (10 min) to 1.2 g of the chlorophosphoramidite (**XIV**) at room temperature and the solution stirred at room temperature for 6 hours. The reaction mixture was poured into 200 ml of ice-cold water. It was then extracted with ethylacetate (3 x 200 ml). The combined organic layer was washed with water. The organic layer was evaporated to dryness to give 3 g (84% yield) of **III** as a white foamy material.

Synthesis of **I** and **II** is conducted according to the same protocol.

The ³¹P-NMR spectrum of **III** has a signal at δ 140 ppm, corresponding to a single P-epimer. In analogy with substitution reactions of **III** involving carbon-, oxygen-, and nitrogen-based nucleophiles (Sun et al. and Carey et al., *supra),* which gave substitution products with overall retention of configuration, **VI** can be formulated as having the structure with Rₚ configuration (Fig. 1). This hitherto unreported nucleoside phosphoramidite **III** is a white solid and is stable when stored dry at 0 - 5°C. The NMR and mass spectral features of **III** are as follows: ³¹P-NMR (CDCl₃) (TMP ext. standard) δ 169 ppm; ¹H-NMR (CDCl₃) δ (ppm) 0.61 (3H, d, J = 6.5 Hz), 1.41 (3H, s, T-CH)₃ 2.42 (2H, m, H-2'), 2.63 (3H, d, ³J_{P-H} = 12 Hz, N-CH₃), 3.37 (1H, dd, J = 10.6, 2.6 Hz, H-5') 3.46 (1H, dd, J = 10.6, 2.6 Hz, H-5'), 3.52 (1H, ddq, J = 6.9, 6.5 Hz, ³J_{P-H} = 2.4 Hz, H-4), 3.76 (6H, s, -OCH₃), 4.08 (1H, m, H-4'), 4.91 (1H, m, H-3'), 5.56 (1H, dd, J = 6.9 Hz, ³J_{P-H(5)} = 1.84 Hz, H-5), 6.41 (1H, dd, J - 6.7, 6.7 Hz, H-1'), 6.85 (4H, m, -Ph), 7.25 (14H, m,-Ph), -76. (1H, s, H-6), 9.1 (1H, s, -NH). FAB-MS (m/z) = 736 (M-H), C₄,H₄₄N₃O₈P.

Oxidative sulfurization of the phosphoramidite **III** with thiolsulfonate (R.I. Chemicals, Costa Mesa, CA) according to Iyer et al., *J. Am. Chem. Soc.* **112**, 1253 (1990), and Iyer et al., *J*. *Org. Chem.* **55,** 4693 (1990) gave the thiophosphoramidates **VII** and **VIII** (90:10, 81% yield) (isomer ratio based on ³¹P-NMR. The NMR and mass spectral features were as follows: **VII**, ³¹P-NMR (CDCl₃) δ (ppm) 79.0; H-NMR (CDCl₃) δ (ppm) 0.78 (3H, d, J = 6.6 Hz, -CH₃) 1.41 (3H, s, T-CH3) 2.55 (2H, m, H-2'), 2.70 (3H, d, ³J_{P-H} = 12.5 Hz, -NCH₃), 3.36 (1H, dd, J = 10.5, 2.3 Hz, H-5'), 3.56 (1H, dd, J = 10.5, 2.2 Hz, H-5') 3.76 (1H, ddq, J = 6.6, 6.1 ³J_{P-H} = 12.3 Hz, H-4), 3.78 (6H, s, -OCH₃), 4.28 (1H, m, H-4'), 5.57 (1H, m, H-3'), 5.62 (1H, dd, J = 6.1 Hz, ³J_{P-H(5)} = 2.8 Hz, H-5), 6.48 (1H, dd, j = 9.0, 5.6 Hz, H-1'), 6.85 (4H, m, -Ph), 7.26 (14H, m, -Ph), 7.62 (1H, s, H-6) 8.90 (1H, s, - NH). FAB-MS (m/z) 769, C₄₁H₄₄N₃O₈PS.

The predominant isomer, **VII** (which is easily separated from **VIII** by flash chromatography), has been assigned the configuration indicated in Fig. 1. The assignment of configurations for **VII** and **VIII** is based on the generally accepted notion that P(III) oxidations proceed with high stereoselectivity and with overall retention of configuration. E.g., Beaucage and Iyer, *Tetrahedron* **48**, 2223 (1992), and Bentrude et al., *J. Am. Chem. Soc.* **111,** 3981 (1989).

### Example 2

### Synthesis of Nucleotide Dimers Using Diastereomerically Enriched Monomer Synthons

Having obtained the nucleoside phosphoramidite **III** in preparative-scale reactions, the stage was set for its use in solid-phase coupling with CPG-bound nucleoside. Thus, contacting a solution of **III** in acetonitrile with CPG-T (10 mmol) for a period of two minutes in the presence of tetrazole as an activator followed by oxidation with the thiolsulfonate resulted in efficient formation of the phosphorothioate dinucleoside with a coupling efficiency of greater than 95% (as evaluated by "trityl yields"). Iyer et al., *J. Am. Chem. Soc., supra,* and Iyer et al., *J. Org. Chem., supra.* Following synthesis, the CPG-bound product was heated with aqueous ammonium hydroxide (28%, 55°C, 1 hr). Examination of the products by reverse-phase HPLC revealed that the dinucleoside phosphorothioate had been formed as a mixture of diastereomers (Rₚ :Sₚ , 40:60). Interestingly, the commonly used cyanoethylphosphate deprotection strategy (28% aq. NH₄OH, 55°C) was found to be sufficient to cleave the chiral phosphate appendage in the phosphite and generate the phosphorothioate. The lack of high stereoselectivity in the formation of is consistent with other reports wherein epimerization of the phosphorous center (in the case of stereoisomerically pure phosphoramidites) is observed when acidic type activators, e.g., tetrazole, are used in conjunction with phosphoramidite methodology in the synthesis of deoxyribonucleoside phosphorothioates. Stec, *supra,* and Beaucage, *supra.*

### Example 3

### Synthesis and Purification of Oligonucleotides

Oligonucleotides are synthesized on a 1 mmol scale following the standard protocol by using an automated synthesizer (*e.g*., Millipore 8700 DNA Synthesizer, Bedford, MA). Where a predominantly Rₚ configuration is desired, the phosphoramidite **III** is used by dissolving it in dry acetonitrile at a concentration of 50 mg/ml. For phosphorothioate oligonucleotides, the iodine oxidation step is replaced by sulftirization with 3H-1,2-benzodithiol-3-one-1,1-dioxide (Beaucage reagent). Iyer et al., *J. Org. Chem.* **55**, 4693 (1990). Two-hour treatment with ammonium hydroxide at room temperature is carried out to cleave the oligomer from the support and to deprotect nucleoside bases. Oligonucleotides are purified by reverse-phase HPLC and/or PAGE, and desalted by using C-1 SEP-PAK cartridges.

### Example 4

### Stereoselective Synthesis of a Mononucleotide Phosphorothioate

Treatment of **VII** and **VIII** with sodium methoxide in methanol at ambient temperature overnight followed by heating with NH₄OH (28% NH₄OH for 1-2 hr at 55°C gave the phosphorothioate in 90% yield with moderate to high stereoselectivity (as monitored by ³¹P-NMR and HPLC). The Rₚ :Sₚ ratio of the phosphorothioate obtained from **VII** was 70:30, whereas the ratio of isomers obtained from **VIII** was 10:90. Configurations were assigned using the criteria reported for dinucleoside phosphorothioates by Iyer et al., *Bioorg. Med. Chem. Lett.* ***4***, 2471 (1994).

### Example 5

### Stereospecific Phosphorothioate Synthesis

Diazabicyclononane (DBU) (296 mg, 1.95 mmol) is dissolved in anhydrous THF (1.5 ml) and added to 3'-O-t-butyl dimethylsilyl thymidine (46 mg, 0.129 mmol) at 0°C for 20 minutes. This solution is added slowly to the solution of **VII** (50 mg, 0.065 mmol) and the contents stirred for 30 minutes at room temperature. The reaction mixture is allowed to warm to room temperature and stirred for 12 h. The solution is evaporated to remove solvent and treated with ammonium hydroxide (28%, 1 ml) and heated for 4 h at 55°C. The solution is evaporated to dryness. Chromatographic purification affords 45 mg (80% yield) of 5'-O-DMT-3'-O-TBDMS TT dimer with Rₚ :Sₚ ratio of 70:30.

## Claims

1. A monomer synthon for the synthesis of oligonucleotides, the monomer synthon having the general structure: 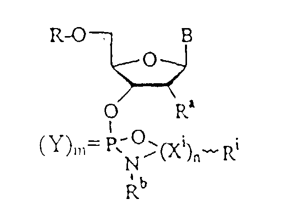 wherein Y is sulfur or oxygen and m=0 or 1, R^{a} and R^{b}, and each Rⁱ are independently H or a C₁-C₂₀ alkyl aryl, heterocyclic or alkoxy group, R is a suitable protecting group, n is 1-3, i is 1-n, Xⁱ is C, O, S, or N, such that if n > 1 the identity of each Xⁱ is independent of the identity of every other Xⁱ and the identity of each substituent Rⁱ is independent of every other Rⁱ, each Rⁱ is covalently bound to the corresponding Xⁱ, the Xⁱ are arranged consecutively such that Xⁱ is bound to the N and Xⁿ is bound to the O, and B is any suitably protected modified or unmodified purine or pyrimidine base, with the proviso, that if B = thymine and R = dimethoxytrityl, R^{a} and R^{b} are not each H.

2. The monomer synthon according to claim 1, wherein R^{a} is H, n is 2 and X¹ and X² are each C and m is 0 or 1.

3. The monomer synthon according to claim, 2, wherein R¹ is methyl, R² is phenyl, R^{a} is H, and R^{b} is methyl.

4. The monomer synthon according to claim 3, wherein m=0 and having Rₚ stereochemical configuration.

5. The monomer synthon according to claim 2, wherein R¹ and R² are each H, R^{a} is H, and R^{b} is methyl.

6. The monomer synthon according to claim 3, wherein m=1 and the monomer synthon is an *anti*-isomer.

7. The monomer synthon according to claim 3, wherein m=1 and the monomer synthon is a *syn*-isomer.

8. A process for synthesizing monomer synthons according to claims 1 to 7, the process comprising reacting the reagent PCl₃ with a second reagent having the structure: 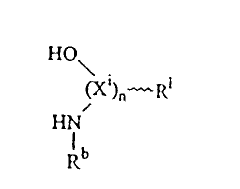 wherein R^{b} and each Rⁱ are independently H or a C₁-C₂₀ alkyl, aryl, heterocyclic or alkoxy group, n is 1-3, i is 1-n, Xⁱ is C, O, S, or N, such that if n > 1 the identity of each Xⁱ is independent of the identity of every other Xⁱ and the identity of each substituent Rⁱ is independent of every other Rⁱ, each Rⁱ is covalently bound to the corresponding Xⁱ, and the Xⁱ are arranged consecutively such that X¹ is bound to the N and Xⁿ is bound to the O, to yield the structure: 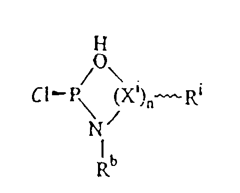 which is then reacted with a nucleoside having a protected 5' hydroxyl and unprotected 3' hydroxyl to yield the monomer synthon and optionally oxidizing the phosphorous of the monomer synthon.

9. The process according to claim 8, wherein for the second reagent n is 2 and X¹ and X² are each C.

10. The process according to claim 9, wherein the second reagent is ephedrine.

11. The process according to claim 10, wherein the ephedrine is a pure stereoisomer of ephedrine.

12. The process according to claim 11, wherein the stereoisomer is selected from the group consisting of (1R,2S)-(-)-ephedrine, (1S,2R)-(-)-ephedrine, (1S,25)-(-)-ephedrine and (1R,2R)-(-)-ephedrine.

13. The process according to claim 9, wherein for the second reagent R¹ and R² are each H, and R^{b} is CH₃.

14. The process according to any one of claims 8 - 13, wherein the oxidation is a thiolative oxidation.

15. The process according to any one of claims 8 - 13, wherein the oxidation is an oxygenation.

16. A process for synthesizing an oligonuclcotide, the process comprising contacting the monomer synthon according to any one of claims 1, 2 or 5, wherein m=0, in the presence of a suitable activating agent with a nascent oligonucleotide having a free 5' hydroxyl to form a phosphite internucleoside linkage and oxidizing the phosphite internucleoside linkage using a suitable oxidizing agent.

17. A process for synthesizing an oligonucleotide having predominantly Sₚ configuration at at least one internucleoside linkage, the process comprising contacting the monomer synthon according to claim 3 wherein m = 0 or claim 4 in the presence of a suitable activating agent with a nascent oligonucleotide having a free 5' hydroxyl to form a phosphite internucleoside linkage and oxidizing the phosphite internucleoside linkage using a suitable oxidizing agent.

18. A process for synthesizing an oligonucleotide, the process comprising contacting the monomer synthon according to anyone of claims 1, 2 or 5, wherein m = 1, in the presence of a suitable activating agent with a nascent oligonucleotide having a free 5' hydroxyl to form a phosphorothioate or phosphodiester internucleoside linkage.

19. A process for synthesizing an oligonucleotide having defined stereochemical configuration at at least one internucleoside linkage, the process comprising contacting the monomer synthon according to claim 3 wherein m=1, or claim 6 or 7 in the presence of a suitable activating agent with a nascent oligonucleotide having a free 5' hydroxyl to form a phosphorothioate or phosphodiester internucleoside linkage.

## Patentansprüche

1. Ein monomeres Synthon zur Synthese von Oligonukleotiden, wobei das Synthon die allgemeine Formel 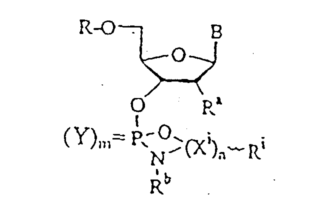 hat,
in der der Rest Y ein Sauerstoff- oder Schwefelatom ist, und m den Wert 0 oder 1 hat, die Reste R^{a} und R^{b} und jeder Rest Rⁱ unabhängig ein Wasserstoffatom oder ein Alkylrest mit 1 bis 20 Kohlenstoffatomen, ein Aryl-, ein heterozyklischer oder ein Alkoxyrest sind, der Rest R eine geeignete Schutzgruppe ist, n einen Wert von 1 bis 3 hat, i einen Wert von 1 - n hat, der Rest Xⁱ ein Kohlenstoff-, Sauerstoff-, Schwefel- oder Stickstoffatom ist, wobei, wenn n > 1 ist, die Identität jedes Rests Xⁱ unabhängig von der jedes anderen Rests Xⁱ ist und die Identität jedes Rests Rⁱ unabhängig von jedem anderen Rest Rⁱ ist, wobei jeder Rest Rⁱ kovalent an den entsprechenden Rest Xⁱ gebunden ist, wobei die Reste Xⁱ aufeinanderfolgend angeordnet sind, derart, dass X¹ and das Stickstoffatom und Xⁿ an das Sauerstoffatom gebunden ist, und der Rest B jede auf geeignete Weise geschützte modifizierte oder unmodifizierte Purin- oder Pyrimidinbase ist, mit der Maßgabe, dass die Reste R^{a} und R^{b} nicht beide Wasserstoffatome sind, wenn der Rest B ein Thymin- und der Rest R ein Dimethoxytritylrest sind.

2. Monomeres Synthon gemäß Anspruch 1, wobei der Rest R^{a} ein Wasserstoffatom ist, n gleich 2 ist und X¹ und X² jeweils Kohlenstoffatome sind und m den Wert 0 oder 1 hat.

3. Monomeres Synthon gemäß Anspruch 2, wobei der Rest R¹ eine Methylgruppe, der Rest R² ein Phenylrest, der Rest R^{a} ein Wasserstoffatom und der Rest R^{b} eine Methylgruppe ist.

4. Monomeres Synthon gemäß Anspruch 3, wobei m gleich 0 ist und das Synthon die stereochemische Rₚ-Konfiguration hat.

5. Monomeres Synthon gemäß Anspruch 2, wobei die Reste R¹ und R² jeweils Wasserstoffatome sind, der Rest R^{a} ein Wasserstoffatom und der Rest R^{b} eine Methylgruppe ist.

6. Monomeres Synthon gemäß Anspruch 3, wobei m den Wert 1 hat und das monomere Synthon ein *anti*-Isomer ist.

7. Monomeres Synthon gemäß Anspruch 3, wobei m den Wert 1 hat und das monomere Synthon ein *syn*-Isomer ist.

8. Verfahren zur Herstellung von monomeren Synthonen gemäß der Ansprüche 1 bis 7, umfassend das Umsetzen von PCl₃ mit einem zweiten Reagenz der allgemeinen Formel 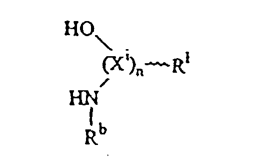 in der die Reste R^{b} und jeder Rest Rⁱ unabhängig ein Wasserstoffatom oder ein Alkylrest mit 1 bis 20 Kohlenstoffatomen, ein Aryl-, ein heterozyklischer oder ein Alkoxyrest sind, n einen Wert von 1 bis 3 hat, i einen Wert von 1 - n hat, der Rest Xⁱ ein Kohlenstoff-, Sauerstoff-, Schwefel- oder Stickstoffatom ist, wobei, wenn n > 1 ist, die Identität jedes Rests Xⁱ unabhängig von der jedes anderen Rests Xⁱ ist und die Identität jedes Rests Rⁱ unabhängig von jedem anderen Rest Rⁱ ist, wobei jeder Rest Rⁱ kovalent an den entsprechenden Rest Xⁱ gebunden ist, und die Reste Xⁱ aufeinanderfolgend derart angeordnet sind, dass X¹ and das Stickstoffatom und Xⁿ an das Sauerstoffatom gebunden ist, unter Bildung einer Verbindung der allgemeinen Formel 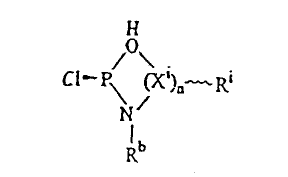 die anschließend mit einem Nukleosid mit einer geschützten 5'-Hydroxylgruppe und einer freien 3'-Hydroxylgruppe umgesetzt wird, unter Bildung des monomeren Synthons, und gegebenenfalls Oxidation des Phosphoratoms des monomeren Synthons.

9. Verfahren gemäß Anspruch 8, wobei in dem zweiten Reagenz n gleich 2 ist und die Reste X¹ und X² beide Kohlenstoffatome sind.

10. Verfahren gemäß Anspruch 9, wobei das zweite Reagenz Ephedrin ist.

11. Verfahren gemäß Anspruch 10, wobei das Ephedrin ein reines Stereoisomer von Ephedrin ist.

12. Verfahren gemäß Anspruch 11, wobei das Stereoisomer ausgewählt ist aus der Gruppe bestehend aus (1R,2S)-(-)-Ephedrin, (1S,2R)-(-)-Ephedrin, (1S,2S)-(-)-Ephedrin und (1 R,2R)-(-)-Ephedrin.

13. Verfahren gemäß Anspruch 9, wobei in dem zweiten Reagenz die Reste R¹ und R² jeweils Wasserstoffatome sind und der Rest R^{b} eine Methylgruppe ist.

14. Verfahren gemäß einem der Ansprüche 8 bis 13, wobei die Oxidation eine oxidative Thiolierung ist.

15. Verfahren gemäß einem der Ansprüche 8 bis 13, wobei die Oxidation eine Oxygenierung ist.

16. Verfahren zur Herstellung eines Oligonukleotids, umfassend: In-Kontaktbringen des monomeren Synthons gemäß einem der Ansprüche 1, 2 oder 5, wobei m gleich 0 ist, in Gegenwart eines geeigneten Aktivierungsmittels mit einem naszierenden Oligonukleotid mit einer freien 5'-Hydroxylgruppe unter Bildung einer Phosphit-Internukleosidbindung und Oxidation der Phosphit-Intemukleosidbindung unter Verwendung eines geeigneten Oxidationsmittels.

17. Verfahren zur Herstellung eines Oligonukleotids mit vorwiegend Sₚ-Konfiguration an mindestens einer Internukleosidbindung, umfassend: In-Kontaktbringen des monomeren Synthons gemäß Anspruch 3, wobei m gleich 0 ist, oder Anspruch 4, in Gegenwart eines geeigneten Aktivierungsmittels mit einem naszierenden Oligonukleotid mit einer freien 5'-Hydroxylgruppe unter Bildung einer Phosphit-Internukleosidbindung und Oxidation der Phosphit-Internukleosidbindung unter Verwendung eines geeigneten Oxidationsmittels.

18. Verfahren zur Herstellung eines Oligonukleotids, umfassend: In-Kontaktbringen des monomeren Synthons gemäß einem der Ansprüche 1, 2 oder 5, wobei m gleich 1 ist, in Gegenwart eines geeigneten Aktivierungsmittels mit einem naszierenden Oligonukleotid mit einer freien 5'-Hydroxylgruppe unter Bildung einer Phosphorthioat- oder Phosphordiester-Internukleosidbindung.

19. Verfahren zur Herstellung eines Oligonukleotids mit einer definierten stereochemischen Konfiguration an mindestens einer Internukleosidbindung, umfassend: In-Kontaktbringen des monomeren Synthons gemäß Anspruch 3, wobei m gleich 1 ist, oder Anspruch 6 oder 7 in Gegenwart eines geeigneten Aktivierungsmittels mit einem naszierenden Oligonukleotid mit einer freien 5'-Hydroxylgruppe unter Bildung einer Phosphorthioat- oder Phosphordiester-Internukleosidbindung.

## Revendications

1. Synthon monomère pour la synthèse d'oligonucléotides, le synthon monomère ayant la structure générale: 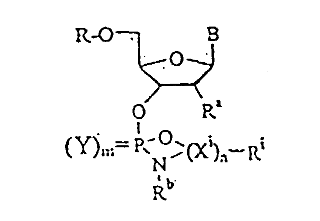 où Y est le soufre ou l'oxygène et m=0 ou 1, R^{a} et R^{b}, et chaque Rⁱ sont indépendamment H ou un groupe alkyle en C₁-C₂₀, aryle, hétérocyclique ou alcoxy, R est un groupe protecteur approprié, n est 1-3, i est 1-n, Xⁱ est C, O, S ou N, de telle sorte que si n > 1 l'identité de chaque Xⁱ est indépendante de l'identité de tous les autres Xⁱ et l'identité de chaque substituant Rⁱ est indépendante de tous les autres Rⁱ, chaque Rⁱ est lié par covalence au Xⁱ correspondant, les Xⁱ sont disposés consécutivement de telle sorte que X¹ est lié au N et Xⁿ est lié au O, et B est n'importe quelle base purine ou pyrimidine modifiée ou non-modifiée, protégée de façon appropriée, avec pour condition que si B = thymine et R = diméthoxytrityle, R^{a} et R^{b} ne sont pas chacun H.

2. Synthon monomère selon la revendication 1, dans lequel R^{a} est H, n est 2 et X¹ et X² sont chacun C et m est 0 ou 1.

3. Synthon monomère selon la revendication 2, dans lequel R¹ est un méthyle, R² est un phényle, R^{a} est H et R^{b} est un méthyle.

4. Synthon monomère selon la revendication 3, dans lequel m=0 et ayant une configuration stéréochimique Rₚ.

5. Synthon monomère selon la revendication 2, dans lequel R¹ et R² sont chacun H, R^{a} est H, et R^{b} est un méthyle.

6. Synthon monomère selon la revendication 3, dans lequel m=1 et le synthon monomère est un isomèreanti.

7. Synthon monomère selon la revendication 3, dans lequel m=1 et le synthon monomère est un isomèresyn.

8. Procédé pour la synthèse de synthons monomères selon les revendications 1 à 7, le procédé comprenant la réaction du réactif PCl₃ avec un second réactif ayant la structure: 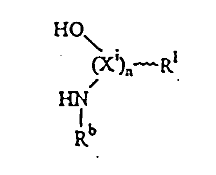 où R^{b} et chaque Rⁱ sont indépendamment H ou un groupe alkyle en C₁-C₂₀, aryle, hétérocyclique ou alcoxy, n est 1-3, i est 1-n, Xⁱ est C, O, S ou N, de telle sorte que si n > 1 l'identité de chaque Xⁱ est indépendante de l'identité de tous les autres Xⁱ et l'identité de chaque substituant Rⁱ est indépendante de tous les autres Rⁱ, chaque Rⁱ est lié par covalence au Xⁱ correspondant, et les Xⁱ sont disposés consécutivement de telle sorte que X¹ est lié au N et Xⁿ est lié au O, pour former la structure: 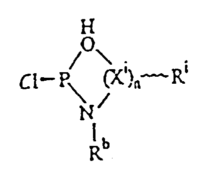 qui est ensuite mise à réagir avec un nucléoside ayant un 5' hydroxyle protégé et un 3' hydroxyle non-protégé pour former le synthon monomère, et en option l'oxydation du phosphore du synthon monomère.

9. Procédé selon la revendication 8, dans lequel pour le second réactif n est 2 et X¹ et X² sont chacun C.

10. Procédé selon la revendication 9, dans lequel le second réactif est l'éphédrine.

11. Procédé selon la revendication 10, dans lequel l'éphédrine est un stéréoisomère pur d'éphédrine.

12. Procédé selon la revendication 11, dans lequel le stéréoisomère est choisi dans le groupe consistant en (1R,2S)-(-)-éphédrine, (1S,2R)-(-)-éphédrine, (1S,2S)-(-)-éphédrine et (1R,2R)-(-)-éphédrine.

13. Procédé selon la revendication 9, dans lequel pour le second réactif R¹ et R² sont chacun H, et R^{b} est CH₃.

14. Procédé selon l'une quelconque des revendications 8-13, dans lequel l'oxydation est une thiolation par voie d'oxydation.

15. Procédé selon l'une quelconque des revendications 8-13, dans lequel l'oxydation est une oxygénation.

16. Procédé pour la synthèse d'un oligonucléotide, le procédé comprenant la mise en contact du synthon monomère selon l'une quelconque des revendications 1, 2 ou 5, où m=0, en présence d'un agent d'activation approprié avec un oligonucléotide naissant ayant un 5' hydroxyle libre pour former une liaison internucléosidique phosphite et l'oxydation de la liaison internucléosidique phosphite au moyen d'un agent d'oxydation approprié.

17. Procédé pour la synthèse d'un oligonucléotide ayant de façon prédominante une configuration Sₚ sur au moins une liaison internucléosidique, le procédé comprenant la mise en contact du synthon monomère selon la revendication 3 où m = 0 ou la revendication 4 en présence d'un agent d'activation approprié avec un oligonucléotide naissant ayant un 5' hydroxyle libre pour former une liaison internucléosidique phosphite et l'oxydation de la liaison internucléosidique phosphite au moyen d'un agent d'oxydation approprié.

18. Procédé pour la synthèse d'un oligonucléotide, le procédé comprenant la mise en contact du synthon monomère selon l'une quelconque des revendications 1, 2 ou 5, où m = 1, en présence d'un agent d'activation approprié avec un oligonucléotide naissant ayant un 5' hydroxyle libre pour former une liaison internucléosidique phosphorothioate ou phosphodiester.

19. Procédé pour la synthèse d'un oligonucléotide ayant une configuration stéréochimique définie sur au moins une liaison internucléosidique, le procédé comprenant la mise en contact du synthon monomère selon la revendication 3 où m = 1, ou la revendication 6 ou 7 en présence d'un agent d'activation approprié avec un oligonucléotide naissant ayant un 5' hydroxyle libre pour former une liaison internucléosidique phosphorothioate ou phosphodiester.
